# FASCICULE DE BREVET EUROPEEN

(11) **EP 2 466 299 B1**
(45) Date de publication et mention de la délivrance du brevet: **21.08.2013**
(21) Numéro de dépôt: 11193869.2
(22) Date de dépôt: 15.12.2011
(51) Int. Cl.: G01N 27/414, G01N 33/00

(54) **Procédé et dispositif de régénération d'un capteur d'hydrogène**
Verfahren und Vorrichtung zur Regenerierung eines Wasserstoffsensors
Method and device for regenerating a hydrogen detector

(30) Priorité: 17.12.2010 FR 1060731
(43) Date de publication de la demande: 20.06.2012
(73) Titulaire: Adixen Vacuum Products, 74000 Annecy (FR)
(72) Inventeur: Patel, Ketan, Monroeville, PA 15146 (US); Pierrejean, Didier, 74000 Annecy (FR)
(74) Mandataire: Croonenbroek, Thomas Jakob

(56) Documents cités:
- EP-A1- 2 251 683
- EP-A2- 0 237 277
- JP-A- 2008 145 128
- US-A1- 2007 229 087
- US-B1- 7 389 675
- FAN ZHIYONG ET AL: "Gate-refreshable nanowire chemical sensors", APPLIED PHYSICS LETTERS, AIP, AMERICAN INSTITUTE OF PHYSICS, MELVILLE, NY, US, vol. 86, no. 12, 17 mars 2005 (2005-03-17) , pages 123510-123510, XP012064774, ISSN: 0003-6951, DOI: 10.1063/1.1883715
- LUNDSTROM I ET AL: "2.11 Hydrogen sensitive MOS structures", VACUUM, PERGAMON PRESS, GB, vol. 27, no. 4, 1 juin 1977 (1977-06-01), pages 245-247, XP025844356, ISSN: 0042-207X, DOI: DOI:10.1016/0042-207X(77)90005-7 [extrait le 1977-06-01]

## Description

La présente invention se rapporte à un procédé de régénération d'un capteur d'hydrogène après utilisation pour la détection de fuite par l'hydrogène au moyen d'un détecteur fonctionnant à basse pression.

Après chaque mesure de fuite, le capteur d'hydrogène doit être régénéré afin de permettre la reproduction fiable des mesures, et conserver une sensibilité élevée pour la détection de la fuite. La régénération du capteur d'hydrogène consiste à neutraliser les ions hydrogène H⁺ piégés sur la grille du transistor contenu dans le capteur. Au cours du processus naturel, ces ions hydrogène H⁺ se combinent avec les molécules d'oxygène O₂ de l'atmosphère. La réaction chimique des ions hydrogène H⁺ avec les molécules d'oxygène O₂ les font sortir de la grille du transistor vers l'atmosphère. Le capteur d'hydrogène est ainsi neutralisé, et se trouve prêt pour le test de fuite suivant. Cependant la régénération du capteur d'hydrogène par ce processus naturel est trop longue et augmente la durée de l'opération de détection, ce qui diminue la fréquence des cycles de détection de fuite. Le rendement de production est donc faible. Le besoin se fait sentir d'un procédé permettant d'accélérer la régénération du capteur d'hydrogène pour augmenter le nombre de cycles.

Toutefois un problème se pose lorsque l'on souhaite effectuer la mesure à basse pression, comprises entre 100 Pa et 5000 Pa, par exemple 1000 Pa. En effet aux basses pressions, il n'y a pas assez de molécules d'oxygène O₂ susceptibles de réagir avec les ions hydrogène H⁺. La régénération du capteur d'hydrogène demandera donc une durée prolongée par rapport au processus naturel, et augmentera d'autant l'intervalle de temps entre deux mesures. Le rendement de production sera donc moindre.

Z. Fan, Applied Physics Letters 86, 123510 (2005), "Gate-refreshable nanowire chemical sensors" décrit un procédé de régénération d'un capteur de NO₂ et NH₃ comprenant un transisteur de type MOS où une tension à la grille du transistor est imposée.

JP2008145128 décrit un capteur d'hydrogène comprenant un transistor de type MOS dont la grille est recouverte d'un catalyseur au palladium.

La présente invention a donc pour but de proposer un procédé de régénération d'un capteur d'hydrogène destiné à la détection de fuite travaillant à basse pression. Le procédé permet une neutralisation complète du capteur dans un temps réduit, moindre que dans le processus naturel connu de l'art antérieur.

L'objet de l'invention est un procédé de régénération d'un capteur d'hydrogène, comprenant un transistor de type MOS dont la grille est recouverte d'un catalyseur au palladium, placé dans une enceinte à basse pression, c'est-à-dire à une pression inférieure à 5000 Pa. Selon ce procédé, après détection d'une fuite, on impose une tension à la grille du transistor au moyen d'un circuit électronique afin de régénérer le catalyseur.

Selon un aspect, la tension est imposée à la grille du transistor de manière pulsée.

Selon un autre aspect, le transistor est chauffé à une température comprise entre 100°C et 250°C.

Selon encore un autre aspect, le circuit électronique comporte un interrupteur permettant de passer du mode « mesure » au mode « régénération »,et vice-versa..

Une autre solution à ce problème pourrait être un apport d'oxygène, mais cette solution est très coûteuse et augmente d'autant le poids du détecteur de fuite. La solution d'imposer une tension à l'aide d'un circuit électronique a pour avantage d'être bon marché, compact, et plus fiable. Elle est facile à développer et facile à installer dans le détecteur de fuite. Le procédé peut être utilisé avec des taux de fuite qui peuvent aller de quelques Pa.m³/s jusqu'à 10⁻⁶ Pa.m³/s.

L'invention a aussi pour objet un module de détection de fuite pour la mise en oeuvre du procédé de régénération du capteur d'hydrogène, comportant :
- Un capteur d'hydrogène comprenant un transistor de type MOS dont la grille est recouverte d'un catalyseur au palladium ;
- Un dispositif de régénération comportant :
   o Un circuit électronique comportant :
      ■ Un générateur DC basses fréquences ;
      ■ Un interrupteur disposé entre le générateur DC basses fréquence et la grille du transistor de type MOS, ledit interrupteur étant apte à imposer une tension à la grille du transistor lorsque l'interrupteur est fermé, et
   o Un module de contrôle pour piloter l'ouverture et la fermeture de l'interrupteur

Selon une variante, l'interrupteur est choisi parmi un relais et un transistor.

D'autres caractéristiques et avantages de la présente invention apparaîtront à la lecture de la description qui suit d'un mode de réalisation, donné bien entendu à titre illustratif et non limitatif, et dans le dessin annexé sur lequel
- la figure 1 illustre un mode de réalisation d'un capteur d'hydrogène,
- la figure 2 illustre un mode de réalisation d'un module de détection de fuites comportant un capteur d'hydrogène et un dispositif de régénération du capteur d'hydrogène,
- la figure 3 illustre l'évolution de la tension V, en volts, en fonction du temps t, en secondes, pendant la régénération du capteur d'hydrogène au moyen du circuit électronique,
- la figure 4 illustre la variation de la tension V, en volts, en sortie du capteur d'hydrogène pendant la mesure et pendant la régénération par le processus naturel en fonction du temps t, en secondes, pour différents taux de fuite.

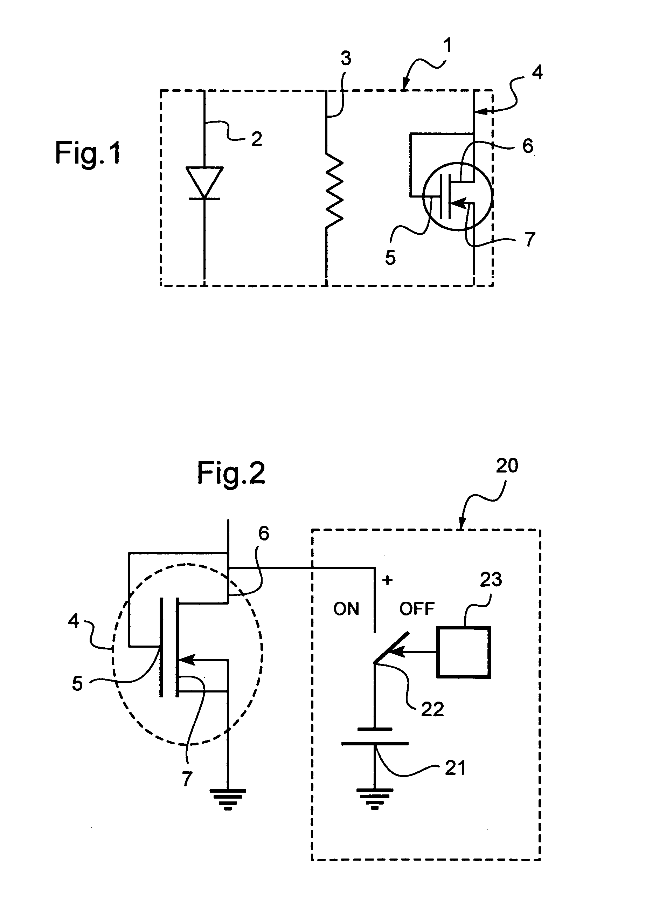

Dans le mode de réalisation illustré sur les figures 1 et 2, un capteur **1** d'hydrogène comporte une diode **2,** une résistance **3** et un transistor **4.**

Le transistor **4** est un transistor à effet de champ de type MOSFET (« Metal Oxide Semiconductor Field Effect Transistor » en anglais, qui se traduit par « transistor à effet de champ à structure métal-oxyde-semiconducteur »). Le transistor **4** est de type »N » et comporte trois électrodes actives, la grille **5** (« gate » en anglais), le drain **6** et la source **7.** Le transistor **4** module le courant qui le traverse au moyen d'un signal appliqué sur la grille **5,** permettant de contrôler la tension **V** entre le drain **6** et la source **7.** La grille **5** est recouverte d'un catalyseur à base de palladium sensible à l'hydrogène. Un courant constant est appliqué au drain **6** du transistor **4** du capteur **1** d'hydrogène afin de polariser le transistor **4.** La quantité d'ions hydrogène H⁺ piégés sur la grille **5,** va modifier la polarisation du transistor ce qui va permettre d'évaluer le flux de molécules d'hydrogène H₂ entrant en contact avec la grille **5.** Lorsqu'il n'y a aucune molécules d'hydrogène H₂ présentes, la tension **V** de sortie du transistor **4** est stable à sa valeur maximale. La valeur de la tension **V** dépend du courant appliqué au drain **6.** Lorsque des molécules d'H₂ arrivent au contact du catalyseur au palladium de la grille **5,** elles sont brisées pour donner des ions H⁺. Les ions H⁺ diffusent à travers le catalyseur au palladium, et se retrouvent piégés sur la grille **5** du transistor **4.** Ceci cause une variation de la résistance drain **6 -** source **7** du transistor **4** qui provoque une baisse de la tension **V** de la grille **5** proportionnelle à la concentration en ions hydrogène H⁺. A partir du calcul de la dérivée en fonction du temps de la baisse de la tension **V,** un taux de fuite peut être calculé. La résistance drain **6 -** source **7** du transistor **4** constitue ainsi la partie sensible du capteur **1** assurant la fonction de mesure de la fuite. En injectant un courant constant dans le drain **6** du transistor **4,** on obtient une valeur de la tension **V** représentant la quantité d'ions H⁺ piégée par la grille **5,** ce qui permet d'évaluer le flux d'hydrogène arrivant au contact de la grille **5.**

La figure 2 représente un module de détection de fuites pour la mise en oeuvre d'un procédé de régénération du capteur **1** d'hydrogène.

Le module de détection de fuites comporte le capteur **1** d'hydrogène et un dispositif de régénération **20.**

Le dispositif de régénération **20** comprend un circuit électronique et un module de contrôle électronique **23.** Le circuit électronique comporte un générateur basses fréquences **21** et un interrupteur **22.**

Le module de contrôle **23** est apte à piloter l'ouverture de l'interrupteur **22** en mode détection et la fermeture de l'interrupteur **22** en mode régénération.

Le module de contrôle **23** comprend un modèle mathématique pour calculer le taux de fuite à partir de la variation de la tension **V** drain **6 -** source **7** du transistor **4** en fonction du temps. Le nombre de molécules d'hydrogène H₂ qui frappe la surface active de la grille **5** du transistor **4** étant proportionnel à la pression, la résistance drain **6 -** source **7** sera donc proportionnelle à la pression absolue d'hydrogène autour du transistor **4.** A une pression résiduelle de 1000 Pa, une monocouche d'atomes d'hydrogène se forme en 25 µs environ.

Une résistance chauffante **3** permet de chauffer le capteur **1** d'hydrogène, à une température de 130°C par exemple, ce qui augmente avantageusement la sensibilité du capteur **1** d'hydrogène. Toutefois la température ne doit pas excéder 250°C, cette limite étant imposée par le silicium du transistor **4.** Des essais ont montré qu'une température de 180°C peut être utilisée sans dommages. A titre d'exemple, la résistance chauffante du capteur **1** d'hydrogène peut avoir une valeur comprise entre 70 Ω et 80 Ω ; à pression atmosphérique, le courant de chauffage sera de 60 à 80 mA, soit une puissance de chauffe de 0,4 W approximativement.

Une diode **2** et la résistance chauffante **3** associée sont utilisées pour asservir la température du capteur **1** d'hydrogène. Pour des raisons de sécurité, on utilise un mélange gazeux composé de 95% d'azote et de 5% d'hydrogène qui n'est mis en contact avec le capteur **1** d'hydrogène qu'une fois la température de 130°C atteinte pour éviter toute dégradation du catalyseur. C'est également pour préserver le catalyseur que le capteur **1** d'hydrogène est utilisé sous très basse pression afin de n'être en contact qu'avec de faible quantité d'hydrogène.

Le circuit électronique impose une tension **U** à la grille **5** du transistor **4** du capteur **1** d'hydrogène. La tension **U** est imposée par le générateur DC basses fréquences **21** associé à l'interrupteur **22** ON-OFF. L'interrupteur **22** peut être par exemple un relais, un transistor ou un système du même type. L'interrupteur **22** est disposé entre le générateur DC basses fréquence **21** et la grille **5** du transistor **4.**

Dans cette configuration, le capteur **1** peut fonctionner en mode « mesure » (interrupteur sur OFF), ce qui permet la détection de fuite, ou en mode « régénération » (interrupteur sur ON) , ce qui permet la neutralisation du capteur. L'interrupteur **22** est ainsi apte à imposer une tension à la grille **5** du transistor **4** lorsque l'interrupteur **22** est fermé.

Ce dispositif permet la régénération du capteur **1** d'hydrogène avec un temps de réponse très court. Ce dispositif est très économique dans la mesure où il ne nécessite qu'un circuit électronique de commande externe.

Une fois la mesure effectuée, il est nécessaire d'éliminer les ions hydrogène H⁺ pris au piège de la grille **5** du transistor **4,** afin que le capteur **1** d'hydrogène soit prêt pour le test de fuite suivant. Pour la régénération du capteur, le module de contrôle pilote la fermeture de l'interrupteur, une tension **U** est ainsi imposée à l'aide du circuit électronique. La tension **U** est imposée par le générateur DC basses fréquences **21** associée à l'interrupteur **22.**

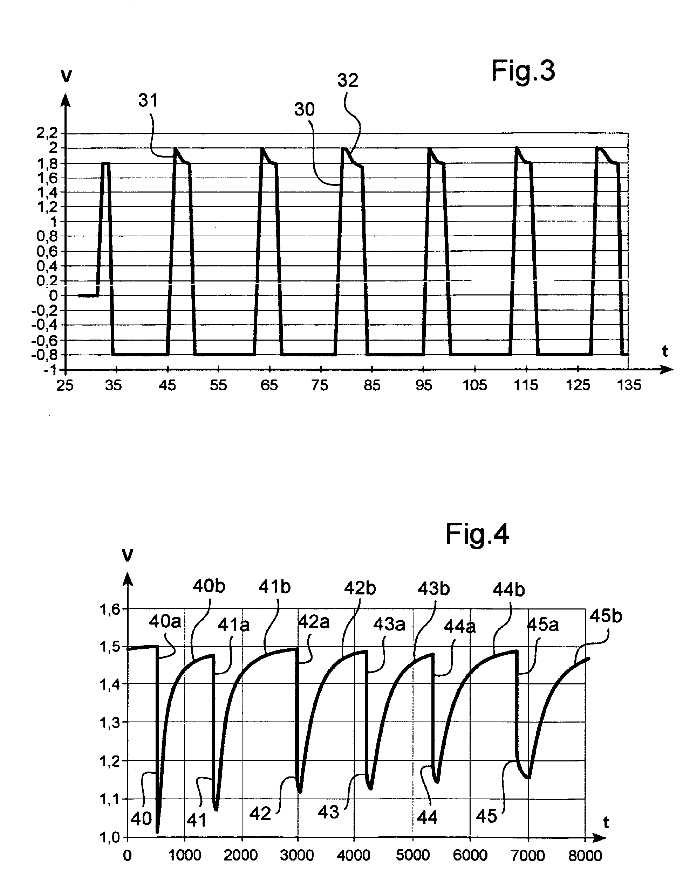

La figure 3 illustre l'évolution de la tension **V** de sortie du transistor **4** en fonction du temps **t.** Au début de la détection de fuite, la grille **5** du transistor **4** est portée à la température requise au moyen de la résistance chauffante **3.** Un courant constant est alors appliqué à la grille **5** du transistor **4** qui produit une tension **V** qui est représentative de son état (partie de courbe **30**). Lorsqu'il n'y a aucune molécules d'hydrogène H₂ présentes, la tension **V** de sortie est stable à sa valeur **maximale 31.** Une fois que les molécules d'hydrogène H₂ atteignent la grille **5** et s'absorbent dans le catalyseur au palladium, la tension **V** commence à décroître (partie de courbe **32**) en fonction de la concentration en ions hydrogène H⁺ absorbés. Le calcul du taux de fuite est effectué à partir de la pente, représentée par la dérivée, de la courbe de tension **V** en fonction du temps **t,** et le résultat de la mesure est affiché.

Lorsque le test de fuite est terminé, les molécules d'hydrogène H₂ cessent de parvenir à la grille **5** du transistor **4,** et la tension **V** commence à croître lentement pour revenir à sa valeur initiale qui est le signe que le capteur **1** se régénère. Cependant comme expliqué précédemment, le processus naturel nécessite une longue durée sous basse pression. C'est à ce moment qu'une tension **U** est imposée à la grille **5** du transistor **4** au moyen du circuit électronique, ce qui accélère la neutralisation des ions hydrogène H⁺ et le retour à la tension d'équilibre **31** du capteur. Cette tension **U** imposée va faire réagir les ions hydrogène H⁺ et neutraliser la grille **5** du transistor **4** du capteur **1** d'hydrogène. Une fois le capteur **1** d'hydrogène totalement régénéré, le module de contrôle **23** pilote l'ouverture de l'interrupteur **22,** on arrête ainsi l'imposition de la tension **U..** Maintenant, le capteur **1** d'hydrogène est de nouveau prêt à effectuer le test de détection de fuite suivant. L'interrupteur **22** permet d'isoler totalement le circuit électronique **20** de la grille **5** du transistor **4** lors de la mesure de fuite.

Une tension **U** est imposée à la grille **5** du transistor **4** de manière pulsée pour obtenir une régénération forcée plus rapide. Le niveau de la tension **U** imposée et l'intervalle de temps entre deux impositions dépend du type de fuite et de l'application souhaitée par le client. Ce procédé permet une régénération par neutralisation du capteur **1** d'hydrogène qui est reproductible et fiable.

La figure 4 illustre les résultats obtenus lors de tests de régénération par le processus naturel pour différentes valeurs du taux de fuite.

Des mesures ont été effectuées, à titre d'exemple, pour les taux de fuite suivants :

| | |
|---|---|
| 1,13.10⁻² Pa.m³.s | courbe **40** |
| 6,08.10⁻³ Pa.m³.s | courbe **41** |
| 4,02.10⁻³ Pa.m³.s | courbe **42** |
| 3,31.10⁻³ Pa.m³.s | courbe **43** |
| 3,04.10⁻³ Pa.m³.s | courbe **44** |
| 2,30.10⁻³ Pa.m³.s | courbe **45** |

Sur la figure 4 on distingue une première partie **40a-45a** sensiblement verticale de la courbe correspondant à la mesure du taux de fuite et une deuxième partie **40b-45b** arrondie de la courbe correspondant à l'étape de régénération par le processus naturel.

Cette régénération par le processus naturel nécessite un temps de l'ordre de 1000 secondes. La régénération forcée au moyen du circuit électronique **20,** telle qu'illustrée sur la figure 3, permet de diviser cette durée par 200.

Bien entendu, la présente invention n'est pas limitée aux modes de réalisation décrits, mais elle est susceptible de nombreuses variantes accessibles à l'homme de l'art sans que l'on s'écarte de l'esprit de l'invention.

## Revendications

1. Procédé de régénération d'un capteur d'hydrogène, comprenant un transistor de type MOS dont la grille est recouverte d'un catalyseur au palladium, placé dans une enceinte de pression inférieure à 5000Pa, **caractérisé en ce que**, après détection d'une fuite, on impose une tension à la grille du transistor au moyen d'un circuit électronique afin de régénérer le catalyseur.

2. Procédé selon la revendication 1, dans lequel la tension est imposée à la grille du transistor de manière pulsée.

3. Procédé selon l'une des revendications 1 et 2, dans lequel le transistor est chauffé à une température comprise entre 100°C et 250°C.

4. Procédé selon l'une des revendications 1 à 3, dans lequel le circuit électronique comporte un interrupteur permettant de passer du mode « mesure » au mode « régénération », et vice-versa.

5. Module de détection de fuite pour la mise en oeuvre du procédé de régénération d'un capteur d'hydrogène selon l'une des revendications1 à 4, comportant :
- un capteur d'hydrogène comprenant un transistor de type MOS dont la grille est recouverte d'un catalyseur au palladium, et
- un dispositif de régénération comprenant :
o un circuit électronique comprenant :
■ un générateur DC basses fréquences,
■ un interrupteur disposé entre le générateur DC basses fréquences et la grille du transistor de type MOS, ledit interrupteur étant apte à imposer une tension à la grille du , transistor lorsque l'interrupteur est fermé, et
o un module de contrôle pour piloter l'ouverture et la fermeture de l'interrupteur.

6. Dispositif selon la revendication 5, dans lequel l'interrupteur est choisi parmi un relais et un transistor.

## Patentansprüche

1. Verfahren zur Regenerierung eines Wasserstoffsensors, der einen MOS-Transistor enthält, dessen Gate mit einem Palladiumkatalysator bedeckt ist, in einem Gehäuse mit einem Druck von weniger als 5000Pa angeordnet, **dadurch gekennzeichnet, dass** nach Erfassung eines Lecks mittels eines elektronischen Schaltkreises eine Spannung an das Gate des Transistors angelegt wird, um den Katalysator zu regenerieren.

2. Verfahren nach Anspruch 1, wobei die Spannung gepulst an das Gate des Transistor angelegt wird.

3. Verfahren nach einem der Ansprüche 1 und 2, wobei der Transistor auf eine Temperatur zwischen 100°C und 250°C erwärmt wird.

4. Verfahren nach einem der Ansprüche 1 bis 3, wobei der elektronische Schaltkreis einen Schalter aufweist, der es ermöglicht, vom Modus « Messung » zum Modus « Regenerierung » und umgekehrt überzugehen.

5. Leckerfassungsmodul zur Durchführung des Verfahrens zur Regenerierung eines Wasserstoffsensors nach einem der Ansprüche 1 bis 4, das aufweist:
- einen Wasserstoffsensor, der einen MOS-Transistor enthält, dessen Gate mit einem Palladiumkatalysator bedeckt ist, und
- eine Regenerierungsvorrichtung, die enthält:
O einen elektronischen Schaltkreis, der enthält:
• einen DC-Niederfrequenzgenerator,
• einen Schalter, der zwischen dem DC-Niederfrequenzgenerator und dem Gate des MOS-Transistors angeordnet ist, wobei der Schalter eine Spannung an das Gate des Transistors anlegen kann, wenn der Schalter geschlossen ist, und
O ein Steuermodul, um das Öffnen und Schließen des Schalters zu steuern.

6. Vorrichtung nach Anspruch 5, wobei der Schalter unter einem Relais und einem Transistor ausgewählt wird.

## Claims

1. Method for regenerating a hydrogen sensor, comprising a transistor of the MOS type whose gate is covered with a palladium catalyst, placed in a enclosure having a pressure less than 5000 Pa, **characterized in that** a voltage is imposed on the gate of the transistor by means of an electronic circuit in order to regenerate the catalyst after a leak has been detected.

2. Method according to Claim 1, wherein the voltage is imposed on the gate of the transistor in a pulsed mode.

3. Method according to one of Claims 1 and 2, wherein the transistor is heated to a temperature of between 100°C and 250°C.

4. Method according to one of Claims 1 to 3, wherein the electronic circuit comprises a switch for changing from the "measurement" mode to the "regeneration" mode, and vice versa.

5. Leak detection module for implementing the method for regenerating a hydrogen sensor according to one of Claims 1 to 4, comprising:
- a hydrogen sensor comprising a transistor of the MOS type whose gate is covered with a palladium catalyst, and
- a regeneration device comprising:
o an electronic circuit comprising:
■ a low-frequency DC generator,
■ a switch arranged between the low-frequency DC generator and the gate of the MOS-type transistor, the said switch being adapted to impose a voltage on the gate of the transistor when the switch is closed, and
o a control module for controlling the closure and opening of the switch.

6. Device according to Claim 5, wherein the switch is selected from among a relay and a transistor.
